# EUROPÄISCHE PATENTSCHRIFT

(11) **EP 3 793 426 B1**
(45) Veröffentlichungstag und Bekanntmachung des Hinweises auf die Patenterteilung: **01.06.2022**
(21) Anmeldenummer: 19722023.9
(22) Anmeldetag: 26.04.2019
(51) Int. Cl.: A61B 5/00, A45D 44/00, G01N 21/359

(54) **BESTIMMUNG DES REDUKTIVEN SCHÄDIGUNGSGRADES VON HAAREN MIT NIR-SPEKTROSKOPIE**
DETERMINATION OF THE DEGREE OF REDUCTIVE DAMAGE OF HAIR WITH NIR SPECTROSCOPY
DÉTERMINATION PAR SPECTROSCOPIE DANS LE PROCHE INFRAROUGE DU NIVEAU DE DÉTÉRIORATION DU CHEVEU LIÉE À LA RÉDUCTION

(30) Priorität: 16.05.2018 DE 102018207560
(43) Veröffentlichungstag der Anmeldung: 24.03.2021
(73) Patentinhaber: Henkel AG & Co. KGaA, 40589 Düsseldorf (DE)
(72) Erfinder: KNUEBEL, Hans Georg, 40219 Düsseldorf (DE); BONNIN, Lucile, 10437 Berlin (DE)
(86) Internationale Anmeldenummer: PCT/EP2019/060734
(87) Internationale Veröffentlichungsnummer: WO 2019/219351

(56) Entgegenhaltungen:
- WO-A1-2018/007357
- WO-A1-2018/007358
- DE-A1-102016 212 202
- US-A1- 2006 281 994

## Beschreibung

"Bestimmung eines reduktiven Schädigungsgrades von Haaren mit NIR-Spektroskopie" Die Erfindung betrifft eine Anordnung zum Bestimmen eines reduktiven Schadens von Haar, ein Verfahren zum Bestimmen des reduktiven Schadens von Haar, und ein Computerprogrammprodukt, welches ausgeführt ist, die Schritte eines solchen Verfahrens auszuführen, wenn es auf einer entsprechenden Anordnung ausgeführt wird.

Verfahren zur Bestimmen eines Schadens von Haar sind aus DE-102016212202-A1, US-2006/281994-A1, WO-2018/007358-A1, und WO-2018/007357-A1 bekannt.

Bei einer Behandlung von Haar mit kosmetischen Produkten kann eine Wirkung des Produkts, z.B. eine Intensität einer Färbung, stark von einer Schädigung des Haars abhängen.

Haar kann durch natürliche oder künstlich herbeigeführte Vorgänge geschädigt werden. Die natürlichen Vorgänge können beispielsweise eine kombinierte (z.B. gleichzeitige) Einwirkung von UV-Licht und Sauerstoff (O₂) auf das Haar aufweisen. Die künstlich herbeigeführten Vorgänge können dabei beispielsweise ein Anwenden von Haarfärbemitteln (auch als Colorationen bezeichnet), ein Blondieren, und/oder ein Erzeugen einer Dauerwelle umfassen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Der hieraus resultierende Schädigungstyp wird als oxidative Schädigung bezeichnet und wird durch die Anwendung von Colorationen, Blondierungen, Dauerwellen und auch durch Umwelteinflüsse (UV + O₂) hervorgerufen. Diese Schädigung wird ursächlich durch die Oxidation der im Haar sehr häufig vorkommenden Aminosäuren Cystin und Cystein zur Cysteinsäure hervorgerufen.

Cystin kann im Haar intermolekulare Disulfidbrücken (auch als S-S-Brücken bezeichnet) ausbilden, so dass das Cystin äußerst wichtig ist für die mechanische Stabilität des Haars.

Die Oxidation dieser Brücken zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenrauigkeit, negativ beeinflusst werden. Auch Ergebnisse kosmetischer Behandlungen, insbesondere schädigender Verfahren, können bereits in einem frühen Schädigungsstadium massiv verändert sein im Vergleich zu einem Ergebnis bei ungeschädigtem Haar.

Neben dieser oxidativen Schädigung ist allerdings auch eine reduktive Schädigung des Haares möglich. Diese tritt bei kosmetischen Verfahren auf, die Reduktionsmittel nutzen. Dies sind z.B. Dauerwellen oder Straightener, die Reduktionsmittel wie z. B. Thioglykolsäure oder Sulfit enthalten.

Diese Inhaltsstoffe dienen der Öffnung der Disulfidbrücken des Cysteins zwecks Umformung der Haare. Dabei werden folgende Schwefel-Spezies ausgebildet: R-S-H (Thiole), R-S-SO₃⁻, (Bunte Salze, nach Sulfit-Behandlung), R-S-S-CH₂COO⁻ (Disulfide mit Thiglykolat-Einheiten, nach Thioglykolat-Behandlung).

In einem akademischen und industriellen Bereich können einem Forscher oder Entwickler eine Vielzahl von physikalischen und chemisch-analytischen Verfahren zur Verfügung stehen, um eine Ermittlung des Schädigungsgrads von Haar durchzuführen. Herkömmlich werden hierbei chromatographische Verfahren verwendet, wie beispielsweise Hochleistungsflüssigkeitschromatographie (HPLC) nach einem aufwändigen sauren hydrolytischen Aufschluss der Haarprobe.

Ungeschädigtes Haar kann typischerweise einen Cysteinsäuregehalt in einem Bereich von etwa 0.5% bis etwa 1% (nach Gewicht) aufweisen. Bei Vorliegen einer Schädigung, beispielsweise infolge mehrfachen Ultrablondierens und/oder anderer Schädigungsmechanismen, kann der Cysteinsäuregehalt auf über 15% (Gew.) ansteigen.

Allerdings sind all diese chromatographischen Verfahren kompliziert und apparativ aufwändig, so dass sie einem Endverbraucher nicht zur Verfügung stehen.

Schädigende kosmetische Behandlungen, beispielsweise Haarcolorationen, Hitzeanwendungen, Dauerwellen oder oxidative Prozeduren wie z.B. Blondieren, und viele andere mehr, werden typischerweise im privaten Bereich oder im Bereich kommerzieller Dienstleistungen am Endverbraucher ausgeführt. Obwohl eine Durchführung eines weiteren schädigenden Verfahrens an vorgeschädigtem Haar zu katastrophalen Ergebnissen bis hin zu einem vollständigen Haarbruch führen kann, bestand in diesem Rahmen bisher keine Möglichkeit, den Grad der Vorschädigung des Haars, beispielsweise quantitativ, zu bestimmen.

Weiterhin gibt es viele verschiedene Haarbehandlungsprodukte am Markt, welche unterschiedliche Haareigenschaften oder-parameter, wie beispielsweise den Glanz, verbessern sollen. In vielen Fällen kennt der Anwender solcher Produkte allerdings den Schaden des Haares nicht. Dies kann dazu führen, dass der Anwender zu in seinem speziellen Fall weniger geeigneten Produkten greift und nach ihrer Anwendung mit deren Wirksamkeit unzufrieden ist.

Deshalb kann eine Ermittlung eines Schadens des Haars von großer Bedeutung sein. Daneben kann es vorteilhaft sein, ein auf individuellen Bedürfnisse abgestimmtes Produkt anzubieten.

Es kann als Aufgabe der vorliegenden Erfindung betrachtet werden, das Bestimmen eines reduktiven Schadens von Haar zu vereinfachen, insbesondere was den apparativen und zeitlichen Aufwand angeht.

Diese Aufgabe wird gelöst mit den Merkmalen der unabhängigen Ansprüche. Weiterbildungen der Erfindung ergeben sich aus den abhängigen Ansprüchen und aus der folgenden Beschreibung.

Der Erfindung liegen unter anderem die folgenden Erkenntnisse zu Grunde: Es wurde erkannt, dass sich die bei einer reduktiven Behandlung von Haar ausgebildeten Schwefel-Spezies schwingungsspektroskopisch charakterisieren lassen.

Da allerdings nach reduktiven Behandlungen aber oftmals noch ein oxidativer Fixierungsschritt erfolgt, ist neben den oben beschriebenen funktionalen Gruppen auch noch ein Anstieg der Cysteinsäure-Einheiten in den Haarproteinen zu beobachten. Die Analyse einer solch komplexen Chemie kann mit schwingungsspektroskopischen Methoden unter Rückgriff auf chemometrische Modelle erfolgen. Die Kalibration des Modells kann dabei durch externe Quantifizierung, z. B. durch HPLC, der oben beschrieben Spezies erfolgen, oder aber auch durch eine Zuweisung eines Haarzustandes der Kalibrierproben auf Basis der erfolgten Haarbehandlung (z. B. 1x leichte Dauerwelle, 2x starke Dauerwelle etc.). Das chemometrische Modell ermittelt dann nicht quantitativ eine chemische Spezies, sondern weist auf Basis der spektralen Daten den unbekannten Haarproben einen ordinalen oder kategorialen Datenwert zu, wobei diese damit dann charakterisiert sind.

Gemäß einem Aspekt ist eine Anordnung zum Bestimmen eines reduktiven Schadens von Haar angegeben. Die Anordnung weist eine Erfassungseinheit zum Erfassen von Haarmerkmalen und eine Auswerteeinheit zum Auswerten der erfassten Haarmerkmale und zum Ermitteln des reduktiven Schadens von Haar basierend auf den erfassten Haarmerkmalen auf. Die Erfassungseinheit enthält einen Nahinfrarotsensor (NIRS) und ist ausgeführt, eine Haarprobe mit elektromagnetischen Wellen im Nahinfrarotbereich bei einer Wellenlänge zwischen 800 und 2500 nm zu bestrahlen und das von der Haarprobe abgegebene Licht zu erfassen. Die Erfassungseinheit ist weiter ausgeführt, ein Spektrum des abgegebenen Lichts zu generieren und der Auswerteeinheit bereitzustellen. Die Auswerteeinheit ist ausgeführt, einen reduktiven Schaden basierend auf dem Spektrum des abgegebenen Lichts in einem Wellenlängenbereich zwischen 1970 und 1980 nm zu ermitteln.

Es wurde erkannt, dass Thiole im Haar bei einer Bestrahlung mit Licht im Nahinfrarotbereich eine Oberschwingung im Bereich 1970 bis 1980 nm haben. Aus dem Spektrum des abgegebeneb Lichts kann dieser Wellenlängenbereich herangezogen werden, um das Ausmaß des reduktiven Schadens zu ermitteln. Die Erfassungseinheit erfasst mindestens diesen Wellenlängenbereich im Spektrum des von der Haarprobe abgegebenen Lichts.

Spektren können grundsätzlich in Transmission oder Reflektion gemessen werden. Bei der Reflektion werden die von einer Probe reflektierten elektromagnetischen Wellen erfasst und bei der Transmission werden die die Probe passierenden/transmittierenden elektromagnetischen Wellen erfasst. Die reflektierten oder passierenden/transmittierenden elektromagnetischen Wellen werden im Sinne dieser Beschreibung als abgegebene elektromagnetische Wellen oder abgegebenes Licht bezeichnet.

Unter einer reduktiven Schädigung von Haar versteht man solche Schäden, welche bei Anwendungen von Reduktionsmitteln oder Reduktionsmittel enthaltenden Behandlungsmitteln an Haar entstehen.

Die Auswerteeinheit kann ein Prozessor oder ein Computer sein, welcher Signalverarbeitungsalgorithmen implementiert und ausführt, um das Spektrum des abgegebenen Lichts auszuwerten und verarbeiten zu können.

Eine Haarprobe kann ein einzelnes Haar sein, enthält aber bevorzugt eine Vielzahl von einzelnen Haaren, um die Intensität des abgegebenen Lichts (oder die Menge des abgegebenen Lichts in Bezug zu dem abgestrahlten Licht) im Vergleich zu einem einzelnen Haar zu erhöhen. Beispielsweise kann Haar zerstörungsfrei untersucht werden, indem die Erfassungseinheit auf das Kopfhaar einer Person gerichtet wird. In anderen Worten kann das Haar untersucht werden, ohne es abschneiden zu müssen.

Unter einem Spektrum von Licht wird vorliegend die Intensität des Lichts über der Frequenz oder Wellenlänge verstanden. Bei dem Spektrum kann es sich um eine kontinuierliche Angabe der Intensität des Lichts in einem Frequenz- oder Wellenlängenbereich von einem unteren Wert zu einem oberen Wert handeln. Diese kontinuierliche Angabe beschreibt den Intensitätswert des Lichts über der Wellenlänge oder Frequenz und hat einen für die chemische Zusammensetzung oder Schaden der Haarprobe charakteristischen Verlauf.

Die Schädigung oder der Schaden kann insbesondere ein Wert sein, beispielsweise ein skalarer Wert, welcher das Ausmaß des Schadens oder die Schadenskategorie (z.B. nicht vorhanden, gering, mittel, stark, sehr stark) anzeigt.

Gemäß einer Ausführungsform ist die Erfassungseinheit ausgeführt, das in Richtung der zu untersuchenden Haarprobe emittierte Licht über den gesamten angegebenen Wellenlängenbereich zwischen 800 und 2500 nm zu emittieren.

Die Haarprobe wird also breitbanding angestrahlt, so dass das abgegebene Licht, die Antwort, ebenfalls breitbandig erfasst werden kann. Es ist denkbar, dass nicht nur das Spektrum alleine in einem schmalen Wellenlängenbereich herangezogen wird, sondern das spektrale Muster ausgehend von dem oben angegebenen schmalen Bereich der Schwingungen von Thiol.

Gemäß einer weiteren Ausführungsform ist die Auswerteeinheit ausgeführt, eine Intensität des abgegebenen Lichts in dem Wellenlängenbereich zwischen 1970 und 1980 nm in Relation zu den darunter und darüber liegenden Wellenlängenbereichen aus dem Wellenlängenbereich des emittierten Lichts zu setzen und den reduktiven Schaden basierend auf dem Muster des Spektrums des abgegebenen Lichts zu ermitteln.

Es wird also nicht alleine der absolute Wert der Intensität des abgegebenen Lichts in dem Wellenlängenbereich zwischen 1970 und 1980 nm herangezogen, sondern dessen Relation zu den Wellenlängenbereichen darunter und darüber. Diese Relation kann anzeigen, ob der Anteil der Thiole in der Haarprobe einen Schwellwert überschreiten oder unterschreiten, so dass aus der Relation über den Schaden der Haarprobe befunden werden kann.

Beispielsweise kann der spektrale Verlauf (die Intensität oder das Muster des Spektrums) zwischen 1970 und 1980 nm in Relation gesetzt werden zu dem spektralen Verlauf zwischen 1900 und 1969 nm einerseits und 1981 und 2050 nm. Die untere Grenze des unteren Wellenlängenbereichs kann auch 1800, 1700, 1600, 1500 nm und weiter in Schritten von 100 nm bis auf 800 nm betragen. Die obere Grenze des oberen Wellenlängenbereichs kann 2100, 2200, 2300, 2400 oder 2500 nm sein.

Durch dieses in Relation Setzen kann ermittelt werden, ob das Spektrum bei 1970 bis 1980 nm ein relatives Maximum, ein relatives Minimum, oder keine signifikante Abweichung von der Intensität in den niedrigeren oder höheren Wellenlängenbereichen hat. Von einem relativen Maximum oder Minimum kann gesprochen werden, wenn die Intensität des abgegebenen Lichts in dem Wellenlängenbereich zwischen 1970 und 1980 nm um mindestens 5% höher ist als die Intensität in den betrachteten Bereichen unterhalb und oberhalb dieses Wellenlängenbereichs. Die angegebene prozentuale Abweichung bezieht sich auf die Intensität in dem untersuchten Wellenlängenbereich zwischen 1970 und 1980 nm. Eine Abweichung kann aber auch als signifikant erachtet werden, wenn sie um mindestens 10%, 15%, 20%, 25% oder mehr abweicht. Dabei kann gelten, dass der reduktive Schaden der Haarprobe umso höher ist, je höher diese Abweichung ist.

Gemäß einer weiteren Ausführungsform ist die Auswerteeinheit ausgeführt, das Spektrum des von der Haarprobe abgegebenen Lichts mit bekannten und verfügbaren Spektren von Haarproben abzugleichen und der Haarprobe basierend auf diesem Abgleich eine Schadenskategorie zuzuordnen.

Die bekannten und verfügbaren Spektren können Teil eines Kalibriermodells sein. Das Kalibriermodell kann beispielsweise basierend auf einer Mehrzahl von Kalibrierhaarproben erstellt werden, wobei für jede einzelne Kalibrierhaarprobe ein Kalibrierspektrum erfasst wird (wie auch oben für das Erzeugen des Spektrums der zu untersuchenden Haarprobe beschrieben) und die Kalibrierhaarprobe mit anderen unabhängigen analytischen Verfahren auf Schäden untersucht, und der damit ermittelte Schaden der Kalibrierhaarprobe zugeordnet wird.

Durch einen Abgleich der Spektren der Kalibrierhaarproben mit dem Spektrum der zu untersuchenden Haarprobe kann der Schaden für letztere ermittelt werden. Bei diesem Abgleich kann die Form des Spektrums und/oder dessen Intensität berücksichtigt werden. Die Berücksichtigung der Form kann auch dann einen Treffer beim Abgleich liefern, wenn die absoluten Werte der chemischen Zusammensetzung der untersuchten Haarprobe und der Kalibrierhaarproben nicht übereinstimmen.

Das Kalibriermodell können in einem lokalen Speicher der Anordnung oder in einer externen Datenspeichereinheit gespeichert sein. Die Erfassungseinheit nimmt zumindest einen Teil eines Spektrums von NIR-Licht, welches mit der Haarprobe gewechselwirkt hat, auf und gleicht dieses Spektrum mit einer Mehrzahl von Kalibrierhaarproben ab. Für diesen Abgleich wird beispielsweise ein Prozessor der Auswerteeinheit verwendet und es wird zumindest ein Teil des Spektrums der untersuchten Haarprobe mit dem spektroskopischen Kalibriermodell zum Ermitteln des reduktiven Schadens des Haars verglichen.

Als nahes Infrarot (NIR) im Sinne dieser Beschreibung werden elektromagnetische Wellen mit einer Wellenlänge zwischen 780 nm und 3000 nm (jeweils inklusive) bezeichnet. Wie an anderer Stelle in diesem Dokument beschrieben, werden aus diesem Wellenlängenbereich bestimmte kleinere Wellenlängenbereiche ausgewählt, um ein Spektrum einer Haarprobe zu erstellen und um aus diesem Spektrum auf den reduktiven Schaden der Haarprobe zu schließen.

Gemäß einer weiteren Ausführungsform ist der Nahinfrarotsensor ein Spektrometer, wobei das Spektrometer ausgeführt ist, basierend auf dem erfassten Licht ein Signalmuster zu erstellen, welches charakteristisch für die Haareigenschaften ist.

Dies bedeutet, dass nicht zwingend absolute Werte für Eigenschaften des Haars bestimmt werden müssen. Vielmehr kann es ausreichen, die erhaltenen Signalmuster des Spektrometers zu nutzen, um eine Produktempfehlung und/oder einen Anwendungshinweis für die Behandlung und/oder Pflege des Haars zu ermitteln. Das Signalmuster kann Teil einer jeden Kalibrierprobe des Kalibriermodells sein. Es kann unabhängig hiervon ermitteln werden, welche Produkte und/oder Anwendungshinweise für Haarproben mit einem bestimmten Signalmuster sinnvoll sind. Den so ermittelten Produkten und/oder Anwendungshinweisen kann dann das entsprechende Signalmuster zugeordnet werden, um auszudrücken, dass sich diese Produkte und/oder Anwendungshinweise für Haar mit diesem Signalmuster eignen und um eine bestimmte Wirkung zu erzielen. Dies bedeutet, dass das Auffinden einer geeigneten Produktempfehlung vereinfacht wird, weil nur die ermittelten Signalmuster der untersuchten Haarprobe mit Signalmustern, die den Produkten und/oder Anwendungs- und Behandlungshinweisen zugeordnet sind, verglichen werden müssen.

Gemäß einer weiteren Ausführungsform weist die Anordnung weiterhin ein Gehäuse und einen Energiespeicher auf, wobei die Auswerteeinheit in dem Gehäuse untergebracht und die Erfassungseinheit mit dem Gehäuse gekoppelt ist, und wobei der Energiespeicher in dem Gehäuse angeordnet ist, um die Auswerteeinheit mit Energie zu versorgen, und um zumindest zeitweise einen autarken Betrieb der Auswerteeinheit ohne Anschluss an eine externe Energiequelle zu ermöglichen.

Dies bedeutet, dass die Auswerteeinheit autark und ohne Zufuhr von Energie von außen wie vorgesehen betrieben werden können. Der Energiespeicher ist bevorzugt ein wiederaufladbarer Energiespeicher. In einem Ausführungsbeispiel ist die Nutzerschnittstelle auch in dem Gehäuse angeordnet und kann von der Energiequelle mit Energie versorgt werden, um ebenfalls autark betrieben zu werden. In einem weiteren Ausführungsbeispiel kann der Energiespeicher auch die Erfassungseinheit mit Energie versorgen.

Das Gehäuse und die Auswerteeinheit können Bestandteil eines persönlichen Geräts eines Nutzers sein. Das persönliche Gerät kann ein portabler Computer in Form eines Smartphones, eines Tablets oder eines anderen Computers sein (diese Einheiten können allgemein als Recheneinheit oder portable Recheneinheit bezeichnet werden). Die Erfassungseinheit kann an die Recheneinheit angeschlossen oder darin integriert sein und wie hierin beschrieben zum Ermitteln des reduktiven Schädigungsgrads von Haaren verwendet werden. In einem Computerprogrammprodukt (z.B. Software oder Anwendung für das persönliche/portable Gerät) werden die erfassten Merkmale des Haars dann in Form von Werten, willkürlichen Einheiten angezeigt oder akustisch ausgegeben. Aus den Parametern können dann (a) Produktempfehlungen für individuell passende Behandlungsprodukte und individuelle Behandlungstipps abgeleitet und/oder (b) der Behandlungserfolg bei einer kosmetischen Behandlung, die das Ziel hat, die gemessen Parameter positiv zu beeinflussen, ermittelt und/oder angezeigt werden.

Die Erfassungseinheit kann eine Schnittstelle (auch: Datenübertragungsverbindung) aufweisen, über welche eine Verbindung mit der Recheneinheit hergestellt wird. Die Recheneinheit kann eine erste Schnittstelle und eine zweite Schnittstelle aufweisen. Die erste Schnittstelle kann als Gegenstück zu der Schnittstelle der Erfassungseinheit ausgeführt sein, also um die Erfassungseinheit an die Recheneinheit anzubinden. Die zweite Schnittstelle kann ausgeführt sein, die Recheneinheit mit einem Datennetz zu verbinden. Diese Verbindungen sind ausgeführt, Informationen in mindestens eine Richtung zu übertragen, bevorzugt in beide Richtungen. Die Verbindung zwischen Erfassungseinheit und Recheneinheit einerseits und die Verbindung zwischen Recheneinheit und einem Zugangspunkt des Datennetzes können drahtgebunden oder drahtlos sein. Drahtgebundene Verbindungen können beispielsweise optische oder elektrische Signale für die Informationsübertragung nutzen. Drahtlose Verbindungen nutzen typischerweise elektromagnetische Wellen für die Signalübertragung, z.B. Funksignale oder auch optische Signale.

Für die Anbindung der Erfassungseinheit an die Recheneinheit können Protokolle verwendet werden, die gemäß den Grundsätzen vermaschter Netze (mesh network) arbeiten. Beispielsweise kann für die Datenübertragung und für die Anbindung der Erfassungseinheit an die Recheneinheit das Thread-Protokoll verwendet werden, welches auf IPv6 aufsetzt. Das Thread-Protokoll wird insbesondere eingesetzt, um automatisierte oder teilautomatisierte Geräte miteinander zu verbinden, in diesem Fall beispielsweise die Erfassungseinheit mit der Recheneinheit.

Die Erfassungseinheit kann in einem Beispiel auf die Recheneinheit strukturell aufgesteckt werden, oder umgekehrt die Recheneinheit auf die Erfassungseinheit. Dies bedeutet, dass die Erfassungseinheit mechanisch an der Recheneinheit oder einem Gehäuse der Recheneinheit befestigt wird. Beispielsweise kann dies durch eine werkzeuglose Montage über eine reversible Verbindung erfolgen. In der aufgesteckten Position kann die Erfassungseinheit mit einer lösbaren kraftschlüssigen oder formschlüssigen Verbindung relativ zu der Recheneinheit gehalten werden. Die Schnittstellen zwischen Erfassungseinheit und Recheneinheit können so angeordnet sein, dass in der aufgesteckten Position eine elektrische Verbindung zwischen Erfassungseinheit und Recheneinheit automatisch hergestellt oder aufgebaut wird.

Die Recheneinheit kann eine Anwendung (oder ein Programm, im Weiteren auch als Computerprogrammprodukt bezeichnet) ausführen, welche Daten von der Erfassungseinheit erhält oder abfragt. Die erhaltenen oder abgefragten Daten werden in der Anwendung verwertet, um einen oder mehrere Ausgabewerte zu ermitteln. Die Daten werden von der Anwendung gemäß der hierin beschriebenen Ansätze verarbeitet und/oder ausgewertet.

Um die Anwendung auszuführen, können Prozessoren (und einer oder mehrere Speicherbausteine) der Recheneinheit verwendet werden. Die Recheneinheit kann aber auch ausgeführt sein, Rechenschritte für das Ausführen der Anwendung auszulagern. So kann die Anwendung beispielsweise die von der Erfassungseinheit erhaltenen oder abgefragten Daten an eine externe Recheneinheit übertragen. Bevor die Daten an die externe Recheneinheit übertragen werden, können sie einer Vorverarbeitung zugeführt werden.

Die externe Recheneinheit kann räumlich entfernt von der Erfassungseinheit und der portablen Recheneinheit angeordnet sein. Die portable Recheneinheit kann über das Datennetz mit der externen Recheneinheit verbunden sein, d.h. in einer Kommunikationsverbindung stehen. Die externe Recheneinheit kann ein einzelner Computer oder Prozessor oder ein Verbund von Computern oder Prozessoren sein. In einem Rechner- oder Prozessorverbund kann die Rechenlast unter verschiedenen Gesichtspunkten auf die einzelnen Bestandteile des Verbunds verteilt werden. Dieser Rechnerverbund kann neben Rechenleistung auch Speicherkapazitäten für die Benutzer bereitstellen und von den Benutzern freigegebene oder gekennzeichnete Daten vorhalten. Damit kann der in der portablen Recheneinheit benötigte Speicherplatz reduziert werden. Auch wird es dem Benutzer erleichtert, eine portable Recheneinheit auszutauschen, weil lokal keine oder fast keine Daten gespeichert sind. Der Rechnerverbund kann als eine Gruppe von vermascht vernetzten Servern ausgestaltet sein.

Gemäß einer weiteren Ausführungsform ist die Auswerteeinheit ausgeführt, die Merkmale von Behandlungsmitteln zur Behandlung von Haar mit den ermittelten Haareigenschaften abzugleichen und eine Wirkung der Behandlungsmittel auf die Haare unter Berücksichtigung der ermittelten Haareigenschaften zu bestimmen.

Dies bedeutet, dass die Behandlungsmittel in Abhängigkeit der ermittelten Haareigenschaften ausgesucht und bestimmt werden. Diese Behandlungsmittel können beispielsweise auf der Nutzerschnittstelle angezeigt oder auf andere Weise ausgegeben werden.

Gemäß einer weiteren Ausführungsform ist die Auswerteeinheit ausgeführt, die ermittelten Haareigenschaften an eine Datenspeichereinheit zu übertragen und Hinweise zur Behandlung des Haars gemäß den ermittelten Haareigenschaften von der Datenspeichereinheit abzufragen.

Bei diesen Hinweisen kann es sich um allgemeine Hinweise (ohne Bezug zu einem bestimmten Behandlungsmittel) betreffend die Behandlung und/oder Pflege von Haar handeln, es kann sich aber auch um Hinweise mit Bezug zu einem bestimmten Behandlungsmittel handeln. Die Hinweise können auch Erklärungen beinhalten, welche Verhaltensweisen welche Eigenschaften des Haars wie beeinflussen.

In der Datenspeichereinheit können Informationen aus Studien sowie Informationen aus Literaturquellen und/oder wissenschaftlichen Veröffentlichungen enthalten sein. Die Auswerteeinheit kann ausgeführt sein, einem Nutzer in Abhängigkeit der erfassten Eigenschaften des Haars einen Auszug aus diesen Informationen auszugeben oder zumindest darauf hinzuweisen.

Gemäß einer weiteren Ausführungsform weist die Anordnung weiterhin eine Nutzerschnittstelle auf und die Auswerteeinheit ist ausgeführt, die Nutzerschnittstelle anzuweisen, die erhaltenen Hinweise zur Behandlung der Haare auszugeben.

Bei der Nutzerschnittstelle kann es sich beispielsweise um ein Display der portablen Recheneinheit handeln, insbesondere um einen sog. Touchscreen, welcher es ermöglicht, Inhalte optisch anzuzeigen und Benutzereingabe über Berührung entgegenzunehmen. Informationen können alternativ oder zusätzlich auch auditiv ausgegeben werden.

Gemäß einer weiteren Ausführungsform ist die Auswerteeinheit ausgeführt, Informationen von einem Nutzer abzufragen und diese Informationen beim Abfragen der Datenspeichereinheit zusätzlich zu berücksichtigen, um von der Datenspeichereinheit Merkmale von Behandlungsmitteln zur Behandlung von Haar gemäß der von dem Nutzer abgefragten Informationen zu erhalten.

Die abgefragten Informationen können mittels eines vorgegebenen Fragebogens erfasst werden, bei welchem einer Aussage durch den Nutzer mehr oder weniger Gewicht beigemessen wird oder auch aus einer von mehreren möglichen Antworten ausgewählt wird. Der vorgegebene Fragebogen kann sich insbesondere mit den Lebensgewohnheiten und außergewöhnlichen Belastungen des Nutzers befassen, zum Beispiel Ernährungsgewohnheiten, Dauer und Qualität des Schlafs, Trinkmenge, Art der Getränke, Verwendung von Genussmitteln (beispielsweise Nikotin, Alkohol), beruflichen Aktivitäten und Freizeitaktivitäten (viel Zeit außerhalb von Gebäuden bei jeglicher Witterung, Aufenthalt in den Bergen, Besuch eines Solariums). Es können auch Alter, Geschlecht und Ethnizität der Benutzer abgefragt sowie für das Abfragen des Behandlungsmitteldatenspeichers herangezogen werden. Auch können die abgefragten Informationen sich auf eine gewünschte oder zu erreichende Eigenschaft des Haars beziehen.

Gemäß einer weiteren Ausführungsform weist die Auswerteeinheit einen lokalen Speicher auf, welcher ausgeführt ist, die von der Datenspeichereinheit abgerufenen Daten zu speichern, bevorzugt persistent zu speichern.

Dies bedeutet, dass die Auswerteeinheit zumindest temporär ihre Funktionen ausführen kann, ohne auf eine dauerhafte Verbindung zu der Datenspeichereinheit aber das Datennetz angewiesen zu sein. Die abgerufenen Daten sind in dem lokalen Speicher gespeichert. Die Daten werden in dem lokalen Speicher so abgespeichert, dass sie bei einem Ausschalten oder außer Betrieb setzen der Auswerteeinheit erhalten bleiben (persistente Speicherung). Es ist möglich, dass die Auswerteeinheit lediglich solche Daten aus der Datenspeichereinheit abruft, welche zu einem momentanen Bild oder zu momentanen Eigenschaften des Haars passen. In einer Ausführungsform können auch solche Daten abgerufen und lokal gespeichert werden, welche zu geringfügig geänderten Eigenschaften des Haars ausgehend von dem aktuellen Zustand passen. Es ist also nicht nötig, alle Daten aus der Datenspeichereinheit abzurufen und in dem lokalen Speicher zu speichern. Vielmehr können gezielt diejenigen Daten oder Informationen aus der Datenspeichereinheit in den lokalen Speicher übertragen werden, welche zu dem erfassten Zustand des Haars passen.

Gemäß einer weiteren Ausführungsform ist die Auswerteeinheit ausgeführt, die ermittelten Haareigenschaften mit einem Zeitstempel betreffend die Ermittlung der Haareigenschaften versehen in dem lokalen Speicher abzuspeichern.

Dies ermöglicht es, Veränderungen des Haars über der Zeit zu beobachten und zu analysieren. Somit können diese Veränderungen auch herangezogen werden, um geeignete nicht-therapeutische Behandlungsmittel und/oder Behandlungshinweise auszugeben. Weiterhin ermöglicht es diese Ausführungsform einem Nutzer, die Veränderungen zu beobachten, um gegebenenfalls das Erreichen von oder das Annähern an selbst gesetzte Ziele feststellen zu können.

Gemäß einer weiteren Ausführungsform ist die Auswerteeinheit ausgeführt, die ermittelten Haareigenschaften über einen längeren Zeitraum umfassend zumindest zwei Vorgänge des Erfassens der Haareigenschaften in dem lokalen Speicher abzuspeichern und wahlweise eine Entwicklung der Haareigenschaften über eine vorgebbare Zeitspanne aus dem lokalen Speicher abzurufen und die Ausgabeeinheit anzuweisen, diese Entwicklung auszugeben.

Gemäß einer weiteren Ausführungsform ist die Auswerteeinheit ausgeführt, die ermittelten Haareigenschaften an die Datenspeichereinheit zu übertragen.

Die ermittelten und übertragenen Haareigenschaften können in der Datenspeichereinheit einer Kennziffer oder einer Kennung eines Nutzers zugewiesen werden, so dass ein Nutzer seine Daten von verschiedenen Geräten einsehen kann. Ebenso hat dieses Vorgehen den Vorteil, dass die Daten eines Nutzers bei Verlust oder Defekt der persönlichen Auswerteeinheit an einer zentralen Stelle gesichert oder hinterlegt sind.

Weiterhin ermöglicht es diese Ausführungsform, Haareigenschaften eines Nutzers über einen längeren Zeitraum zu erfassen und deren Veränderung zu beobachten und gegebenenfalls mit den Empfehlungen für nicht-therapeutische Behandlungsmittel und/oder Behandlungshinweisen zu verknüpfen.

Gemäß einer weiteren Ausführungsform ist die Anordnung ausgeführt, Anweisungen für die Bedienung der Erfassungseinheit visuell auf der Ausgabeeinheit und/oder akustisch über einen Lautsprecher auszugeben. Dies kann insbesondere hilfreich sein, wenn das Haar eines Benutzers erstmalig und umfassend erfasst wird, um einen Überblick über den Zustand des Haars zu erlangen.

Gemäß einer weiteren Ausführungsform ist die Ausgabeeinheit ausgeführt, Informationen über ein Behandlungsmittel, z.B. eine Produktbezeichnung, Informationen betreffend Inhaltsstoffe und/oder Zusammensetzung eines Behandlungsmittels und/oder Anwendungshinweise zur nicht-therapeutischen Behandlung des Haars auszugeben.

Somit wird es einem Nutzer ermöglicht, sich selbst ein Bild über ein Behandlungsmittel in seiner Gesamtheit zu machen. Außerdem können dem Nutzer Anwendungshinweise bezogen auf ein Behandlungsmittel oder unabhängig hiervon gegeben werden. Die Anwendungshinweise können sich auf gewünschtes und/oder ungewünschtes Verhalten beziehen.

Gemäß einer weiteren Ausführungsform ist die Nutzerschnittstelle ausgeführt, nach dem Ausgeben von Merkmalen eines Behandlungsmittels eine Eingabe von einem Nutzer entgegen zu nehmen und basierend auf dieser Eingabe eine Aktion betreffend das angezeigte Behandlungsmittel zu veranlassen.

Die Aktion kann sich beispielsweise darauf beziehen, dass dem Nutzer ein Behandlungsmittel zum Kauf angeboten wird und der Nutzer über eine Eingabe den Kauf in die Wege leiten kann. Neben dem Kauf von Behandlungsmitteln können dem Nutzer auch weitergehende Informationen zum käuflichen Erwerb angeboten werden. Diese weitergehenden Informationen können sich auf detailliertere Behandlungs- und Anwendungshinweise beziehen. Das Programm empfängt beispielsweise die Anfrage, dass der Nutzer das Behandlungsmittel erwerben möchte, speichert die Anfrage und/oder übermittelt die Anfrage an ein Handelsunternehmen, welches die Behandlungsmittel vertreibt. Der Nutzer wird durch das Computerprogramm aufgefordert, seine persönlichen Daten (Adresse, Bankinformationen, Versendungspräferenz, etc.) über die Eingabeeinheit einzugeben.

Alternativ kann dem Benutzer ausgegeben werden, wo, beispielsweise in einem Drogeriemarkt, in einem Friseursalon, in einer Apotheke, etc. in seiner Nähe, er das ausgegebene Behandlungsmittel vor Ort erwerben kann.

Immer mehr Kunden wünschen sich ein individuell auf ihre Bedürfnisse abgestimmtes Produkt. Entsprechend kann dem Nutzer ein individuell für ihn hergestelltes Behandlungsprodukt empfohlen und ein Bestellvorgang, beispielsweise durch Aufrufen einer Internetseite eines Herstellers von individuellen Haarbehandlungsmitteln, eingeleitet werden.

Dabei kann es sich um ein speziell für den einen Nutzer hergestelltes Behandlungsmittel oder ein sogenanntes "mass customized" Produkt handeln. Bei einem "mass customized" Produkt kann durch Variation von wenigen, aus Kundensicht jedoch entscheidenden, Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurators abläuft. Dieser Konfigurator hilft dem Nutzer bei der Auswahl der Merkmale/Inhaltsstoffe und weist ihn auf die zulässigen/unzulässigen Merkmalskombinationen hin, wobei letztere dann nicht ausgewählt werden können.

Bei Haarbehandlungsmitteln umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Schädigungsgrad/Dehnungsgrad/etc. ungeeigneter Inhaltsstoffe vermieden werden. Umgekehrt kann die Auswahl für den ermittelten Schädigungsgrad/Dehnungsgrad/etc. geeigneter Inhaltsstoffe durch den Produktkonfigurator vorgegeben oder vorgeschlagen werden.

Ebenso ist es möglich, ein individuelles Haarbehandlungsmittel vor Ort, das heißt zum Beispiel in einem Friseursalon oder an einem Verkaufspunkt von Haarbehandlungsmitteln, wie beispielsweise ein Drogeriemarkt, mittels einer Mischvorrichtung, vorzugsweise einer intelligenten Mischvorrichtung (Smart Mixer), herzustellen.

Gemäß einem weiteren Aspekt ist ein Verfahren zum Bestimmen eines reduktiven Schadens von Haar angegeben. Das Verfahren weist die folgenden Schritte auf: Bestrahlen einer Haarprobe mit elektromagnetischen Wellen im Nahinfrarotbereich bei einer Wellenlänge zwischen 800 und 2500 nm; Erfassen des von der Haarprobe abgegebenen Lichts; Erzeugen eines Spektrums des abgegebenen Lichts; Ermitteln des reduktiven Schadens von Haar basierend auf dem Spektrum des abgegebenen Lichts in einem Wellenlängenbereich zwischen 1970 und 1980 nm.

Dieses Verfahren entspricht der Funktion der oben beschriebenen Anordnung. Die Verfahrensschritte entsprechen dabei den Funktionen der Anordnung und werden an dieser Stelle nicht erneut beschrieben. Jedenfalls wird für Details zu den Verfahrensschritten auf die obige Beschreibung der Anordnung verwiesen. Die übrigen Funktionen der Anordnung können selbstverständlich auch als Verfahrensschritte implementiert werden.

Gemäß einer Ausführungsform weist das Verfahren folgenden Schritt auf: Abgleichen des Spektrums des abgegebenen Lichts mit bekannten und verfügbaren Spektren von Haarproben und Zuordnen einer Schadenskategorie an die Haarprobe basierend auf diesem Abgleich.

Gemäß einem weiteren Aspekt ist ein Verfahren zum Ermitteln eines Behandlungsmittels basierend auf dem bestimmten reduktiven Schaden von Haar angegeben. Dieses Verfahren weist die folgenden Schritte auf: Heranziehen des bestimmten reduktiven Schadens von Haar und Auswählen eines Behandlungsmittels für Haar basierend auf dem ermittelten reduktiven Schaden und Ausgeben von Informationen über das ausgewählte Behandlungsmittel.

Gemäß einer Ausführungsform weist das Verfahren zum Ermitteln eines Behandlungsmittels folgenden Schritt auf: Auswählen des Behandlungsmittels ausgehend von einem gewünschten Behandlungsergebnis.

Das Behandlungsmittel wird in Abhängigkeit des ermittelten oder bestimmten reduktiven Schadens und unter Berücksichtigung einer gewünschten Wirkung, beispielsweise gewünschter Eigenschaften der Haare nach der Behandlung, ausgesucht oder ermittelt. Bei der gewünschten Wirkung kann es sich um eine von einem Benutzer vorgegebene Wirkung oder einen gewünschten Zustand des Haars handeln. Hierbei kann es hilfreich sein, dass jedem Behandlungsmittel eine oder mehrere Schadensarten zugeordnet werden, bei der eine Anwendung möglich ist, und auch eine Wirkung, welche das Behandlungsmittel bei der entsprechenden Schadensart hat. Somit kann über einen einfachen Abgleich der Schadensart und der gewünschten Wirkung das passende Behandlungsmittel ermittelt werden.

Gemäß einem weiteren Aspekt ist ein Computerprogrammprodukt angegeben, welches ausgeführt ist, das Verfahren wie hierin beschrieben auszuführen, wenn es auf einer Anordnung wie ebenfalls hierin beschrieben ausgeführt wird.

Das Computerprogrammprodukt ermöglicht die Kontrolle und Nachverfolgung der Ergebnisse durch Darstellung (z.B. graphisch) der Messergebnisse im Verlauf der Zeit. Anhand der erzielten Ergebnisse gibt das Computerprogrammprodukt individuelle Behandlungs- und Produkttipps. Die Qualität der Behandlungs- und Produkttipps kann dadurch verbessert werden, dass der Benutzer zusätzlich Fragen zu seinem Haarzustand, seinen Ernährungsgewohnheiten, seinem generellen Gesundheitszustand und weiteren Verhaltensweisen beantwortet, die das Computerprogrammprodukt entsprechend verarbeiten kann. Dazu werden nicht nur z.B. Literaturdaten zu Grunde gelegt, sondern auch der Behandlungserfolg anderer Nutzer des Computerprogrammprodukts, insbesondere Behandlungserfolge anderer Nutzer, die zumindest einen ähnlichen Haarzustand aufweisen.

Die mittels des Fragebogens erfassten Daten können verwendet werden, um eine Entwicklung des Zustands der Haare des Nutzers unter den gegebenen Umständen, also den von dem Nutzer eingegebenen Daten, zu analysieren. Diese Entwicklung kann mit der Entwicklung anderer Nutzer verglichen werden. Hieraus kann gefolgert werden, ob sich während einer Behandlung mit einem bestimmten Produkt die Entwicklung von Nutzern mit ähnlichen oder gleichen Eingaben im Fragebogen gleicht oder von Nutzern mit anderen Eingaben abweicht.

So kann beispielsweise auf den Einfluss eines bestimmten Faktums auf den Erfolg der Behandlung geschlossen werden. Zeigt beispielsweise die Entwicklung einer Schadensart bei mehreren rauchenden Personen mit einem bestimmten Zigarettenkonsum (z.B. zehn Zigaretten pro Tag) eine signifikante Abweichung von der Entwicklung der gleichen Schadensart bei Nichtrauchern, mag gefolgert werden, dass Rauchen sich auf den bestimmten Parameter in einer zu beziffernden Weise auswirkt. Alternativ kann gefolgert werden, dass für Raucher ein anderes Produkt oder eine andere Behandlung empfehlenswert ist.

Die von dem Nutzer eingegebenen Daten können somit für eine globale Analyse verwendet werden, um den Erfolg einer Behandlung und die Wirksamkeit eines Produkts unter verschiedenen Bedingungen überwachen und ggf. Änderungen der Behandlung und/oder des Produkts empfehlen zu können.

Ausführungsbeispiele der Erfindung sind in den Figuren dargestellt und werden im Folgenden näher erläutert. Dabei zeigt:
- Fig. 1: eine schematische Darstellung einer Anordnung zum Ermitteln eines reduktiven Schadens von Haar gemäß einem Ausführungsbeispiel;
- Fig. 2: eine schematische Darstellung eines Datenträgers gemäß einem weiteren Ausführungsbeispiel;
- Fig. 3: eine schematische Darstellung von Schritten eines Verfahrens gemäß einem weiteren Ausführungsbeispiel;
- Fig. 4: eine schematische Darstellung einer Erfassungseinheit für eine Anordnung gemäß einem weiteren Ausführungsbeispiel.

In der folgenden ausführlichen Beschreibung wird auf die beigefügten Zeichnungen Bezug genommen, die einen Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder funktionale oder logische Änderungen vorgenommen werden können, ohne von dem Schutzumfang der vorliegenden Erfindung abzuweichen. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche sowie Äquivalente hiervon definiert.

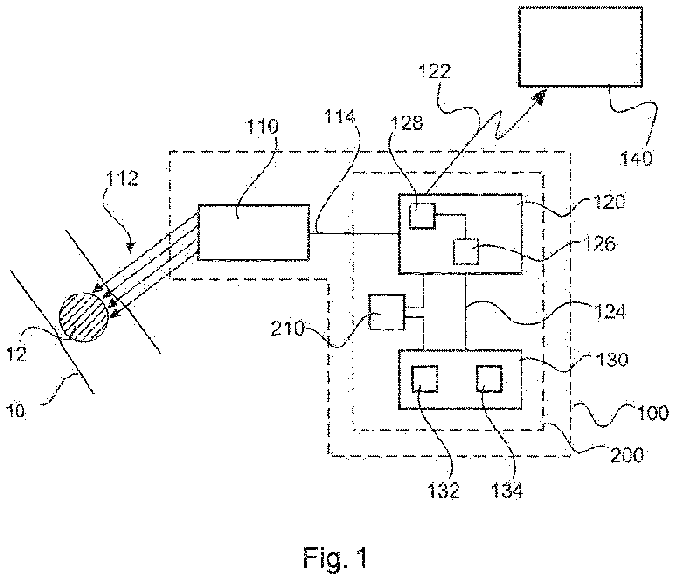

Fig. 1 zeigt eine Anordnung 100 zum Ermitteln eines reduktiven Schadens von Haar. Die Anordnung 100 weist eine Erfassungseinheit 110, eine Auswerteeinheit 120 und eine Nutzerschnittstelle 130 auf. Die Erfassungseinheit 110 ist ausgeführt, Eigenschaften von Haar zu erfassen. Hierzu emittiert die Erfassungseinheit 110 Licht in einem Wellenlängenbereich zwischen 800 nm und 2500 nm in Richtung eines zu untersuchenden Bereichs 12 des Analyseobjekts 10 (beispielsweise von menschlichem Haar) und erfasst abgegebenes Licht insbesondere in dem Wellenlängenbereich zwischen 1970 und 1980 nm (inklusive dieser beiden Werte). Das von dem zu untersuchenden Bereich 12 abgegebene Licht wird von der Erfassungseinheit 110 aufgenommen und erlaubt Rückschlüsse auf den reduktiven Schaden des Haars, weil die bei reduktiver Schädigung des Haars auftretenden Thiole in dem erfassten Wellenlängenbereich eine Oberschwingung haben.

Die Erfassungseinheit 110 weist eine geeignete Quelle elektromagnetischer Wellen auf. Diese Quelle ist ein Lichtemitter oder Laseremitter und kann auch als Strahlungsquelle bezeichnet werden und ist an oder in der Erfassungseinheit 110 angeordnet. Die Strahlungsquelle kann so an oder in der Erfassungseinheit 110 angeordnet sein, dass beim Emittieren der elektromagnetischen Wellen 112 die Strahlungsquelle einen vorgegebenen Abstand zu dem zu untersuchenden Bereich 12 einnimmt, insbesondere wenn die Erfassungseinheit 110 auf den zu untersuchenden Bereich gestellt wird. Der Abstand der Strahlungsquelle von dem zu untersuchenden Bereich kann variabel sein und mittels Aktuatoren oder manuell verändert werden.

Die Erfassungseinheit 110 ist über eine Datenübertragungsverbindung 114 mit der Auswerteeinheit 120 verbunden. Die Datenübertragungsverbindung 114 kann eine unidirektionale oder bidirektionale Datenübertragung zwischen der Erfassungseinheit 110 und der Auswerteeinheit 120 ermöglichen. So liefert die Erfassungseinheit 110 Signale betreffend die erfassten Merkmale des Haars an die Auswerteeinheit 120, wohingegen die Auswerteeinheit 120 Steuerbefehle an die Erfassungseinheit 110 liefern kann, wobei die Steuerbefehle vorgeben, wie die Erfassungseinheit 110 arbeitet. Im Falle einer unidirektionalen Datenübertragungsverbindung 114, welche lediglich eine Datenübertragung von der Erfassungseinheit 110 an die Auswerteeinheit 120 ermöglicht, können Steuerparameter über Eingabeelemente (Knöpfe, Schalter, Drehregler, etc., nicht gezeigt) an der Erfassungseinheit 110 vorgegeben werden. Die Erfassungseinheit 110 weist gegebenenfalls Anzeigeelemente (nicht gezeigt) auf, welche einen Status der Erfassungseinheit oder die eingestellten Steuerparameter anzeigen. Alternativ kann die Erfassungseinheit 110 die eingestellten Steuerparameter auch an die Auswerteeinheit 120 übertragen, wo diese wahlweise angezeigt werden können.

Die Auswerteeinheit 120 weist einen Prozessor 126 und einen lokalen Speicher 128 auf. Die Auswerteeinheit 120 empfängt Signale betreffend die Merkmale des untersuchten Bereiches 12 und ermittelt basierend auf diesen Merkmalen eine Empfehlung betreffend eine nicht-therapeutische Behandlung des untersuchten Haars. Die nicht-therapeutische Behandlung kann Empfehlungen zu Behandlungsmitteln und/oder Behandlungshinweisen oder Anwendungshinweisen für das jeweils untersuchte Haar enthalten. Behandlungshinweise und Anwendungshinweise werden im Kontext dieser Beschreibung als Synonyme verwendet und beziehen sich auf Hinweise für die nicht-therapeutische Behandlung des untersuchten Bereichs (Haars) 12 unter Verwendung von ausgewählten Behandlungsmitteln oder auch ohne eine Verwendung von Behandlungsmitteln. Behandlungshinweise können insbesondere die Anwendung eines Behandlungsmittels enthalten, oder auch von dem Nutzer vorzunehmende oder zu unterlassende Maßnahmen. So können die Behandlungshinweise beispielsweise einen Hinweis auf erwünschtes oder unerwünschtes Verhalten nach der Anwendung eines Behandlungsmittels enthalten. Zum Ermitteln einer zu empfehlenden nicht-therapeutischen Behandlung können die erfassten Merkmale des untersuchten Bereiches 12 mit Anwendungsgebieten, Wirkungen und Anwendungshinweisen von Behandlungsmitteln und/oder Behandlungshinweisen abgeglichen werden. Informationen über die Behandlungsmittel und/oder Behandlungshinweise können in einer Datenspeichereinheit 140 hinterlegt sein.

Die Datenspeichereinheit 140 kann außerhalb und räumlich getrennt von der Auswerteeinheit 120 angeordnet sein. Die Auswerteeinheit 120 kann über ein Datennetz 122 auf die Datenspeichereinheit 140 zugreifen und Informationen über die dort hinterlegten Behandlungsmittel und/oder Behandlungshinweise abrufen. Diese abgerufenen Informationen werden durch die Auswerteeinheit 120 mit den erfassten Merkmalen des zu untersuchenden Bereiches 12 abgeglichen, um zutreffende Empfehlungen für die nicht-therapeutische Behandlung des untersuchten Haars zu ermitteln. In anderen Worten bedeutet das, dass die Datenspeichereinheit unter Verwendung der ermittelten Haareigenschaften abgefragt wird. Aus der Datenspeichereinheit kann zunächst eine Vielzahl von hinterlegten Informationen abgerufen werden, um diese dann unter Verwendung der ermittelten Haareigenschaften und ggf. von Behandlungszielen danach zu filtern, welche der Behandlungsmittel und/oder Behandlungshinweise einschlägig sind. Hierzu können die Daten aus dem Datenspeicher in einen flüchtigen Arbeitsspeicher geladen werden. Alternativ können aber bereits beim Abrufen der Informationen aus dem Datenspeicher die ermittelten Haareigenschaften herangezogen werden, um nur die einschlägigen Informationen aus dem Datenspeicher abzurufen. Für die Zwecke dieser Beschreibung können diese beiden Varianten als in Ihrer Wirkung gleichwertig verstanden werden.

Das Datennetz 122 kann ein öffentliches Datenübertragungsnetz sein, welches abschnittsweise leitungsgebundene oder leitungslose Übertragungsabschnitte aufweist. Beispielsweise mag die Auswerteeinheit 120 eine drahtlose Verbindung zu einem Zugangspunkt (nicht gezeigt) zu dem Datennetz 122 aufbauen, um eine entsprechende Verbindung zu der Datenspeichereinheit 140 aufbauen zu können.

Die Nutzerschnittstelle 130 ist mit der Auswerteeinheit 120 über die Datenübertragungsverbindung 124 verbunden. Die Nutzerschnittstelle 130 weist eine Eingabeeinheit 132 und eine Ausgabeeinheit 134 auf. Die Eingabeeinheit 132 ermöglicht es einem Nutzer, Parameter für die Arbeitsweise und Konfiguration der Auswerteeinheit 120, der Erfassungseinheit 110 und/oder der Nutzerschnittstelle 130 vorzugeben. Die Eingabeeinheit 132 kann Informationen über verschiedene Schnittstellen aufnehmen: eine Tastatur, eine Maus, ein berührungsempfindliches Display oder über ein Mikrofon (sog. Sprachsteuerung). Es ist denkbar, dass jegliche Schnittstelle genutzt wird, über welche ein menschlicher Nutzer mit einer Recheneinheit kommunizieren und Daten eingeben oder übergeben kann. Die Ausgabeeinheit 134 kann ein Display oder eine sonstige Anzeigeeinheit sein, welche einem Nutzer visuelle Informationen ausgibt. Auch kann die Ausgabeeinheit 134 über einen Lautsprecher verfügen, über welchen akustische Informationen ausgegeben werden können. Visuelle Informationen können auf einer berührungsempfindlichen Ausgabeeinheit ausgegeben werden, so dass die Ausgabeeinheit es auch ermöglicht, dass ein Nutzer hierüber Eingaben vornehmen kann.

Die Auswerteeinheit 120 weist einen Prozessor 126 und einen lokalen Speicher 128 auf. Der Prozessor 126 führt Instruktionen aus, um seine vorgesehene Funktion oder Funktionen auszuführen. In dem lokalen Speicher 128 können die von der Erfassungseinheit 110 erfassten Merkmale des Haars oder die zugehörigen Signale oder Werte abgespeichert werden.

Es ist ein besonderer Aspekt dieses Ausführungsbeispiels, dass die Erfassungseinheit 110 mit einer Auswerteeinheit 120 und einer Nutzerschnittstelle 130 betrieben werden können, welche in einem portablen Gerät eines Nutzers oder Verbrauchers implementiert sind. Damit ist es besonders einfach möglich, eine Erfassungseinheit 110, welche fortgeschrittene Analyse- und Untersuchungsmöglichkeiten für das Haar eines menschlichen Nutzers ermöglicht, mit einem tragbaren computerisierten Datenverarbeitungsgerät zu koppeln. Das tragbare Datenverarbeitungsgerät kann beispielsweise ein Smartphone oder ein Tablet sowie ein Heimcomputer sein. Die Erfassungseinheit 110 kann mittels einer definierten Schnittstelle mit dem tragbaren Datenverarbeitungsgerät mechanisch, elektrisch und signaltechnisch verbunden oder gekoppelt werden.

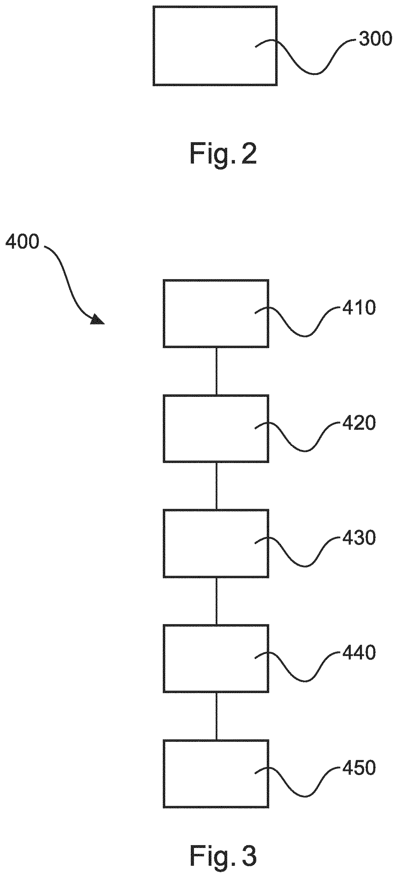

Fig. 2 zeigt einen Datenträger 300. Auf dem Datenträger ist ein Computerprogrammprodukt abgelegt, welches ausgeführt ist, auf einer portablen Recheneinheit 120 ausgeführt zu werden und einen Prozessor 126 der portablen Recheneinheit anzuweisen, die folgenden Schritte auszuführen (vgl. auch die Verfahrensschritte in Fig. 3): Bestrahlen (410) einer Haarprobe mit elektromagnetischen Wellen im Nahinfrarotbereich bei einer Wellenlänge zwischen 800 und 2500 nm; Erfassen (420) des von der Haarprobe abgegebenen Lichts; Erzeugen (430) eines Spektrums des abgegebenen Lichts; Ermitteln (440) des reduktiven Schadens von Haar basierend auf dem Spektrum des abgegebenen Lichts in einem Wellenlängenbereich zwischen 1970 und 1980 nm.

Der Datenträger 300 kann magnetische, optische oder elektrische Speichertechniken (oder Kombinationen hiervon) nutzen, um die Anweisungen des Computerprogrammprodukts in einer maschinenlesbaren Form vorzuhalten. Diese Anweisungen können verwendet werden, um unmittelbar von dem Prozessor 126 einer portablen Recheneinheit 120 (der Auswerteeinheit 120 aus dem Ausführungsbeispiel der Fig. 1) ausgeführt zu werden. Alternativ können die Anweisungen verwendet werden, um das Computerprogrammprodukt in einen internen Speicher der portablen Recheneinheit 120 zu laden, um dann von dort ausgeführt zu werden. Bei diesem internen Speicher kann es sich beispielsweise um den lokalen Speicher 128 aus der Fig. 1 handeln.

Der Datenträger 300 kann ein mobiler und/oder tragbarer Datenspeicher sein. Alternativ kann das Computerprogrammprodukt auch über ein Datennetz geladen werden, indem von einer portablen Recheneinheit über das Datennetz auf den Datenträger 300 zugegriffen wird, um die Computerprogrammprodukt über das Datennetz zu laden. Das Computerprogrammprodukt kann über ein Datennetz auf ein portables Gerät eines Nutzers geladen und auf dem portablen Gerät zur Verwendung durch den Nutzer installiert werden.

Ergänzend zu Fig. 2 ist in Fig. 3 ein Verfahren 400 mit den folgenden Schritten gezeigt (diese Schritte entsprechen den Funktionen des Computerprogrammprodukts): Bestrahlen (410) einer Haarprobe mit elektromagnetischen Wellen im Nahinfrarotbereich bei einer Wellenlänge zwischen 800 und 2500 nm; Erfassen (420) des von der Haarprobe abgegebenen Lichts; Erzeugen (430) eines Spektrums des abgegebenen Lichts; Ermitteln (440) des reduktiven Schadens von Haar basierend auf dem Spektrum des abgegebenen Lichts in einem Wellenlängenbereich zwischen 1970 und 1980 nm; und Abgleichen (450) des Spektrums des abgegebenen Lichts mit bekannten und verfügbaren Spektren von Haarproben und Zuordnen einer Schadenskategorie an die Haarprobe basierend auf diesem Abgleich.

Das Computerprogrammprodukt enthält Anweisungen, welche den Prozessor 126 der portablen Recheneinheit 120 anweisen, diese Schritte 410 bis 450 auszuführen.

Selbstverständlich kann das Verfahren 400 oder dessen Schritte 410 bis 450 in Einklang mit einem der Ausführungsbeispiele der Anordnung 100, wie mit Bezug zu Fig. 1 und der übrigen Beschreibung dargestellt, modifiziert werden. Dies bedeutet, dass die hierin beschriebenen Funktionen der Anordnung 100 oder einer ihrer Komponenten, insbesondere der Auswerteeinheit 120, als Schritt des Verfahrens 400 implementiert werden können. Es wird darauf verzichtet, die Funktionen der Auswerteeinheit an dieser Stelle zu wiederholen. Vielmehr wird der Fachmann erkennen, dass und wie diese Funktionen als Verfahrensschritte implementiert werden.

Die verschiedenen Verfahrensschritte sowie die Komponenten der Anordnung können durch ein oder mehrere Schaltkreise realisiert sein. In einer Ausführungsform ist ein "Schaltkreis" als jegliche Einheit zu verstehen, die eine Logik implementiert, und die sowohl Hardware, Software, Firmware oder eine Kombination daraus sein kann. Somit kann ein "Schaltkreis" in einer Ausführungsform ein hart-verdrahteter Logik-Schaltkreis oder ein programmierbarer Logik-Schaltkreis sein, wie beispielsweise ein programmierbarer Prozessor, z.B. ein Mikroprozessor oder ein FPGA-Baustein (field programmable gate array, programmierbares Logikgatter). Unter einem "Schaltkreis" kann auch ein Prozessor zu verstehen sein, der Software ausführt, z.B. jegliche Art von Computer-Programm, etwa ein Computer-Programm in Programmiercode für eine virtuelle Maschine (abgegrenzte Laufzeitumgebung, Virtual Machine), wie z.B. ein Java-Computer-Programm. Unter einem "Schaltkreis" kann in einer Ausführungsform jegliche Art der Implementierung der im Weiteren beschriebenen Funktionen zu verstehen sein.

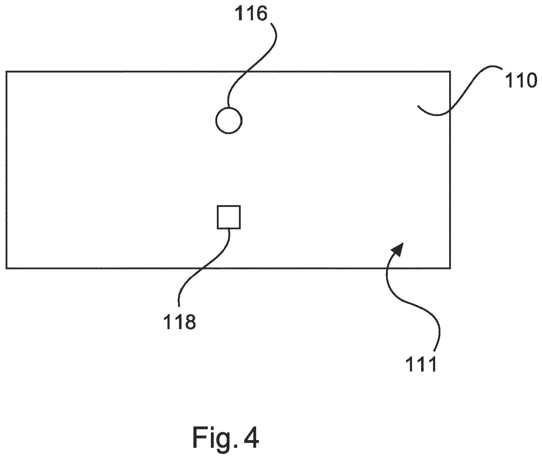

Fig. 4 zeigt eine schematische Darstellung einer Erfassungseinheit 110. Die Erfassungseinheit weist eine Oberfläche 111 auf, an welcher ein Lichtemitter 116 und ein Spektrometer 118 gezeigt sind. Der Lichtemitter 116 ist kreisförmig dargestellt und das Spektrometer 118 ist viereckig dargestellt.

Von der Erfassungseinheit 110 ist diejenige Oberfläche zu sehen, welche bei einem Erfassungsvorgang dem Haar eines Nutzers zugewandt ist. In anderen Worten emittiert der Lichtemitter 116 die Lichtstrahlen aus der Zeichenebene heraus auf einen Betrachter zu. Wird das Haar eines menschlichen Benutzers mit Licht (z.B. Laser) bestrahlt, wird ein Teil dieses Lichts in Abhängigkeit der chemischen Zusammensetzung des Haars abgegeben.

Der Prozessor 126 (Fig. 1) kann Steuerfunktionen implementierten und Steuerbefehle an den Lichtemitter 116 ausgeben. So kann der Prozessor 126 den Lichtemitter ansteuern, um Licht einer gewissen Intensität, Wellenlänge und/oder spektralen Verteilung (diese können als Parameter des Lichts bezeichnet werden) auszugeben.

Die Auswerteeinheit 120 mit dem Prozessor 126 (Fig. 1) empfängt auch die Signale des Spektrometers 118 und kann basierend auf diesen Signalen das untersuchte Haar klassifizieren. In anderen Worten sind die von dem Spektrometer 118 gelieferten Signale kennzeichnend für das untersuchte Haar. Diese Signale können auch als Signalmuster bezeichnet werden und können genutzt werden, um eine Produktempfehlung und/oder Anwendungshinweise zu ermitteln und auszugeben.

Es ist denkbar, dass einem Produkt und/oder einem Anwendungshinweis ein typisches Signalmuster zugeordnet wird, bei dem das Produkt und oder der Anwendungshinweis sinnvollerweise auf das untersuchte Haar aufgebracht werden kann, um ein gewünschtes Behandlungsergebnis zu erzielen. Dieses zugeordnete Signalmuster der Produkte und/oder der Anwendungshinweise kann mit dem tatsächlichen Signalmuster von der Erfassungseinheit verglichen werden. Ab einem gewissen Grad an Übereinstimmung des von dem Spektrometer erfassten oder gelieferten Signalmusters mit dem den Produkten und/oder Anwendungshinweisen zugeordneten Signalmuster können dann die entsprechenden Produkte und/oder Anwendungshinweise ausgegeben werden. Die Signale können auf qualitative Ähnlichkeit (entsprechen sich die Formen oder Verläufe der Signale) und/oder quantitative Ähnlichkeit (haben die Signale für ähnliche Eingangswerte, d.h. Licht, ähnliche Ausgangswerte, d.h. abgegebenes Licht) untersucht werden.

Es ist auch denkbar, dass in Abhängigkeit von Benutzereingaben ein Faktor ermittelt wird, der auf das von dem Spektrometer erfasste Signal angewendet wird, bevor dieses Eingangssignal mit den Signalmustern der Produkte oder Anwendungshinweisen verglichen wird. Dies hat den Vorteil, dass ein Korrekturfaktor auf das erfasste Signal angewandt werden kann, um die Genauigkeit der Produktempfehlungen und/oder Anwendungshinweise für einen bestimmten Nutzer zu verbessern.

### LISTE DER BEZUGSZEICHEN

- 10: Analyseobjekt
- 12: zu untersuchender Bereich
- 100: Anordnung zum Ermitteln von Eigenschaften von Haar
- 110: Erfassungseinheit
- 111: Oberfläche
- 112: elektromagnetische Wellen
- 114: Datenübertragungsverbindung
- 116: Lichtemitter
- 118: Spektrometer
- 120: Auswerteeinheit
- 122: Datennetz
- 124: Datenübertragungsverbindung
- 126: Prozessor
- 128: lokaler Speicher
- 130: Nutzerschnittstelle
- 132: Eingabeeinheit
- 134: Ausgabeeinheit
- 140: Datenspeichereinheit
- 200: Gehäuse
- 210: Energiespeicher
- 300: Datenträger
- 400: Verfahren
- 410 - 450: Verfahrensschritte

## Patentansprüche

1. Anordnung (100) zum Bestimmen eines reduktiven Schadens von Haar, die Anordnung aufweisend:
eine Erfassungseinheit (110) zum Erfassen von Haarmerkmalen;
eine Auswerteeinheit (120) zum Auswerten der erfassten Haarmerkmale und zum Ermitteln des reduktiven Schadens von Haar basierend auf den erfassten Haarmerkmalen;
wobei die Erfassungseinheit (110) einen Nahinfrarotsensor, NIRS, enthält;
wobei die Erfassungseinheit (110) ausgeführt ist, eine Haarprobe mit elektromagnetischen Wellen im Nahinfrarotbereich bei einer Wellenlänge zwischen 800 und 2500 nm zu bestrahlen und das von der Haarprobe abgegebene Licht zu erfassen;
wobei die Erfassungseinheit (110) ausgeführt ist, ein Spektrum des abgegebenen Lichts zu generieren und der Auswerteeinheit (120) bereitzustellen;
wobei die Auswerteeinheit (120) ausgeführt ist, einen reduktiven Schaden basierend auf dem Spektrum des abgegebenen Lichts in einem Wellenlängenbereich zwischen 1970 und 1980 nm zu ermitteln.

2. Anordnung (100) nach Anspruch 1,
wobei die Erfassungseinheit (110) ausgeführt ist, das in Richtung der zu untersuchenden Haarprobe emittierte Licht über den gesamten angegebenen Wellenlängenbereich zwischen 800 und 2500 nm zu emittieren.

3. Anordnung (100) nach Anspruch 1 oder 2,
wobei die Auswerteeinheit (120) ausgeführt ist, eine Intensität des abgegebenen Lichts in dem Wellenlängenbereich zwischen 1970 und 1980 nm in Relation zu den darunter und darüber liegenden Wellenlängenbereichen aus dem Wellenlängenbereich des emittierten Lichts zu setzen und den reduktiven Schaden basierend auf dem Muster des Spektrums des abgegebenen Lichts zu ermitteln.

4. Anordnung (100) nach einem der voranstehenden Ansprüchen,
wobei die Auswerteeinheit (120) ausgeführt ist, das Spektrum des von der Haarprobe abgegebenen Lichts mit bekannten und verfügbaren Spektren von Haarproben abzugleichen und der Haarprobe basierend auf diesem Abgleich eine Schadenskategorie zuzuordnen.

5. Anordnung (100) nach einem der voranstehenden Ansprüche,
wobei der Nahinfrarotsensor ein Spektrometer (118) ist, wobei das Spektrometer ausgeführt ist, basierend auf dem erfassten Licht ein Signalmuster zu erstellen, welches charakteristisch für die Haareigenschaften ist.

6. Anordnung (100) nach einem der voranstehenden Ansprüche,
weiterhin aufweisend ein Gehäuse (200) und einen Energiespeicher (210), wobei die Auswerteeinheit (120) in dem Gehäuse untergebracht und die Erfassungseinheit (110) mit dem Gehäuse gekoppelt ist, und wobei der Energiespeicher in dem Gehäuse (200) angeordnet ist, um die Auswerteeinheit (120) mit Energie zu versorgen, und um zumindest zeitweise einen autarken Betrieb der Auswerteeinheit ohne Anschluss an eine externe Energiequelle zu ermöglichen.

7. Anordnung (100) nach einem der voranstehenden Ansprüche,
wobei die Auswerteeinheit (120) ausgeführt ist, die Merkmale von Behandlungsmitteln zur Behandlung von Haar mit den ermittelten Haareigenschaften abzugleichen und eine Wirkung der Behandlungsmittel auf die Haare unter Berücksichtigung der ermittelten Haareigenschaften zu bestimmen.

8. Anordnung (100) nach einem der voranstehenden Ansprüche,
wobei die Auswerteeinheit (120) ausgeführt ist, die ermittelten Haareigenschaften an eine Datenspeichereinheit (140) zu übertragen und Hinweise zur Behandlung des Haars gemäß den ermittelten Haareigenschaften von der Datenspeichereinheit abzufragen.

9. Anordnung (100) nach Anspruch 8,
weiterhin aufweisend eine Nutzerschnittstelle (130),
wobei die Auswerteeinheit (120) ausgeführt ist, die Nutzerschnittstelle anzuweisen, die erhaltenen Hinweise zur Behandlung der Haare auszugeben.

10. Anordnung (100) nach einem der Ansprüche 8 oder 9,
wobei die Auswerteeinheit (120) ausgeführt ist, Informationen von einem Nutzer abzufragen und diese Informationen beim Abfragen der Datenspeichereinheit (140) zusätzlich zu berücksichtigen, um von der Datenspeichereinheit (140) Merkmale von Behandlungsmitteln zur Behandlung von Haar gemäß der von dem Nutzer abgefragten Informationen zu erhalten.

11. Verfahren (400) zum Bestimmen eines reduktiven Schadens von Haar, aufweisend:
Bestrahlen (410) einer Haarprobe mit elektromagnetischen Wellen im Nahinfrarotbereich bei einer Wellenlänge zwischen 800 und 2500 nm;
Erfassen (420) des von der Haarprobe abgegebenen Lichts;
Erzeugen (430) eines Spektrums des abgegebenen Lichts;
Ermitteln (440) des reduktiven Schadens von Haar basierend auf dem Spektrum des abgegebenen Lichts in einem Wellenlängenbereich zwischen 1970 und 1980 nm.

12. Verfahren (400) nach Anspruch 11, weiter aufweisend:
Abgleichen (450) des Spektrums des abgegebenen Lichts mit bekannten und verfügbaren Spektren von Haarproben und Zuordnen einer Schadenskategorie an die Haarprobe basierend auf diesem Abgleich.

13. Verfahren zum Ermitteln eines Behandlungsmittels basierend auf dem in Anspruch 11 oder 12 bestimmten reduktiven Schaden von Haar, aufweisend die folgenden Schritte:
Heranziehen des bestimmten reduktiven Schadens von Haar;
Auswählen eines Behandlungsmittels für Haar basierend auf dem ermittelten reduktiven Schaden und Ausgeben von Informationen über das ausgewählte Behandlungsmittel.

14. Verfahren nach Anspruch 13, weiter aufweisend:
Auswählen des Behandlungsmittels ausgehend von einem gewünschten Behandlungsergebnis.

15. Computerprogrammprodukt, welches ausgeführt ist, das Verfahren nach einem der Ansprüche 11 bis 14 auszuführen, wenn es auf einer Anordnung nach einem der Ansprüche 1 bis 10 ausgeführt wird.

## Claims

1. An arrangement (100) for determining reductive damage to hair, the arrangement comprising:
a detection unit (110) for detecting hair characteristics;
an evaluation unit (120) for evaluating the detected hair characteristics and for determining the reductive damage to hair based on the detected hair characteristics;
wherein the detection unit (110) contains a near-infrared sensor, NIRS;
wherein the detection unit (110) is designed to irradiate a hair sample with electromagnetic waves in the near-infrared range at a wavelength between 800 and 2500 nm and to detect the light emitted by the hair sample;
wherein the detection unit (110) is designed to generate a spectrum of the emitted light and to provide said spectrum to the evaluation unit (120);
wherein the evaluation unit (120) is designed to determine reductive damage based on the spectrum of the emitted light in a wavelength range between 1970 and 1980 nm.

2. The arrangement (100) according to claim 1,
wherein the detection unit (110) is designed to emit the light emitted in the direction of the hair sample to be examined over the entire indicated wavelength range between 800 and 2500 nm.

3. The arrangement (100) according to claim 1 or 2,
wherein the evaluation unit (120) is designed to set an intensity of the emitted light in the wavelength range between 1970 and 1980 nm in relation to the wavelength ranges below and above the wavelength range of the emitted light and to determine the reductive damage based on the pattern of the spectrum of the emitted light.

4. The arrangement (100) according to one of the preceding claims,
wherein the evaluation unit (120) is designed to compare the spectrum of the light emitted by the hair sample with known and available spectra of hair samples and to assign a damage category to the hair sample based on that comparison.

5. The arrangement (100) according to one of the preceding claims,
wherein the near-infrared sensor is a spectrometer (118), wherein the spectrometer is designed to create a signal pattern based on the detected light, which pattern is characteristic of the hair properties.

6. The arrangement (100) according to one of the preceding claims,
further comprising a housing (200) and an energy store (210), wherein the evaluation unit (120) is accommodated in the housing and the detection unit (110) is coupled to the housing, and wherein the energy store is arranged in the housing (200) in order to supply the evaluation unit (120) with energy, and to allow at least temporarily autonomous operation of the evaluation unit without said unit being connected to an external energy source.

7. The arrangement (100) according to one of the preceding claims,
wherein the evaluation unit (120) is designed to compare the characteristics of treatment agents for treating hair with the determined hair properties and to determine an effect of the treatment agents on the hair, taking into account the determined hair properties.

8. The arrangement (100) according to one of the preceding claims,
wherein the evaluation unit (120) is designed to transmit the determined hair properties to a data storage unit (140) and to request information on the treatment of the hair according to the determined hair properties from the data storage unit.

9. The arrangement (100) according to claim 8,
further comprising a user interface (130),
wherein the evaluation unit (120) is designed to instruct the user interface to output the information obtained on the treatment of the hair.

10. The arrangement (100) according to one of claims 8 or 9,
wherein the evaluation unit (120) is designed to request information from a user and to also take this information into account when requesting information from the data storage unit (140) in order to obtain characteristics of treatment agents for treating hair from the data storage unit (140) according to the information requested from the user.

11. A method (400) for determining reductive damage to hair, comprising:
irradiating (410) a hair sample with electromagnetic waves in the near-infrared range at a wavelength between 800 and 2500 nm;
detecting (420) the light emitted by the hair sample;
generating (430) a spectrum of the emitted light;
determining (440) the reductive damage to hair based on the spectrum of the emitted light in a wavelength range between 1970 and 1980 nm.

12. The method (400) according to claim 11, further comprising:
comparing (450) the spectrum of the emitted light with known and available spectra of hair samples and assigning a damage category to the hair sample based on that comparison.

13. A method for determining a treatment agent based on the reductive damage to hair determined in claim 11 or 12, comprising the following steps:
referring to the specific reductive damage to hair;
selecting a hair treatment agent based on the determined reductive damage and outputting information about the selected treatment agent.

14. The method according to claim 13, further comprising:
selecting the treatment agent starting from a desired treatment outcome.

15. A computer program product which is designed to carry out the method according to one of claims 11 to 14 when said method is carried out on an arrangement according to one of claims 1 to 10.

## Revendications

1. Système (100) permettant de déterminer un endommagement lié à la réduction de cheveux, le système présentant :
une unité de détection (110) permettant de détecter des caractéristiques de cheveux ;
une unité d'évaluation (120) permettant d'évaluer les caractéristiques de cheveux détectées et de déterminer l'endommagement lié à la réduction de cheveux sur la base des caractéristiques de cheveux détectées ;
dans lequel l'unité de détection (110) comprend un capteur proche infrarouge, NIRS ;
dans lequel l'unité de détection (110) est configurée pour irradier un échantillon de cheveux avec des ondes électromagnétiques dans le domaine du proche infrarouge à une longueur d'onde entre 800 et 2 500 nm et pour détecter la lumière émise par l'échantillon de cheveux ;
dans lequel l'unité de détection (110) est configurée pour générer un spectre de la lumière émise et pour le fournir à l'unité d'évaluation (120) ;
dans lequel l'unité d'évaluation (120) est configurée pour déterminer un endommagement lié à la réduction sur la base du spectre de la lumière émise dans une plage de longueurs d'onde entre 1 970 et 1 980 nm.

2. Système (100) selon la revendication 1,
dans lequel l'unité de détection (110) est configurée pour émettre la lumière émise en direction de l'échantillon de cheveux à examiner sur toute la plage de longueurs d'onde indiquée entre 800 et 2 500 nm.

3. Système (100) selon la revendication 1 ou 2,
dans lequel l'unité d'évaluation (120) est configurée pour régler une intensité de la lumière émise dans la plage de longueurs d'onde entre 1 970 et 1 980 nm par rapport aux plages de longueurs d'onde inférieures et supérieures à partir de la plage de longueurs d'onde de la lumière émise et pour déterminer l'endommagement lié à la réduction sur la base du modèle du spectre de la lumière émise.

4. Système (100) selon l'une des revendications précédentes,
dans lequel l'unité d'évaluation (120) est configurée pour comparer le spectre de la lumière émise par l'échantillon de cheveux avec des spectres connus et disponibles d'échantillons de cheveux et pour attribuer une catégorie d'endommagement à l'échantillon de cheveux sur la base de ladite comparaison.

5. Système (100) selon l'une des revendications précédentes,
dans lequel le capteur proche infrarouge est un spectromètre (118), le spectromètre étant configuré pour créer un modèle de signal sur la base de la lumière détectée, lequel est caractéristique des propriétés de cheveux.

6. Système (100) selon l'une des revendications précédentes,
présentant en outre un boîtier (200) et un accumulateur d'énergie (210), l'unité d'évaluation (120) étant logée dans le boîtier et l'unité de détection (110) étant couplée au boîtier, et l'accumulateur d'énergie étant disposé dans le boîtier (200) pour alimenter en énergie l'unité d'évaluation (120) et pour permettre un fonctionnement autonome de l'unité d'évaluation sans connexion à une source d'énergie externe, au moins temporairement.

7. Système (100) selon l'une des revendications précédentes,
dans lequel l'unité d'évaluation (120) est configurée pour comparer les caractéristiques d'agents de traitement pour le traitement de cheveux avec les propriétés de cheveux déterminées et pour déterminer un effet des agents de traitement sur les cheveux en tenant compte des propriétés de cheveux déterminées.

8. Système (100) selon l'une des revendications précédentes,
dans lequel l'unité d'évaluation (120) est configurée pour transmettre les propriétés de cheveux déterminées à une unité de stockage de données (140) et pour demander des indications concernant le traitement des cheveux selon les propriétés de cheveux déterminées à l'unité de stockage de données.

9. Système (100) selon la revendication 8,
présentant en outre une interface utilisateur (130),
dans lequel l'unité d'évaluation (120) est configurée pour instruire à l'interface utilisateur de sortir les indications reçues concernant le traitement des cheveux.

10. Système (100) selon l'une des revendications 8 ou 9,
dans lequel l'unité d'évaluation (120) est configurée pour demander des informations à un utilisateur et pour également prendre en compte lesdites informations lors de la demande à l'unité de stockage de données (140) afin d'obtenir, de l'unité de stockage de données (140), des caractéristiques d'agents de traitement pour le traitement de cheveux selon les informations demandées à l'utilisateur.

11. Procédé (400) permettant de déterminer un endommagement lié à la réduction de cheveux, présentant :
l'irradiation (410) d'un échantillon de cheveux avec des ondes électromagnétiques dans le domaine du proche infrarouge à une longueur d'onde entre 800 et 2 500 nm ;
la détection (420) de la lumière émise par l'échantillon de cheveux ;
la génération (430) d'un spectre de la lumière émise ;
la détermination (440) de l'endommagement de cheveux lié à la réduction sur la base du spectre de la lumière émise dans une plage de longueurs d'onde entre 1 970 et 1 980 nm.

12. Procédé (400) selon la revendication 11, présentant en outre :
la comparaison (450) du spectre de la lumière émise avec des spectres connus et disponibles d'échantillons de cheveux et l'attribution d'une catégorie d'endommagement à l'échantillon de cheveux sur la base de ladite comparaison.

13. Procédé permettant de déterminer un agent de traitement sur la base de l'endommagement lié à la réduction de cheveux déterminé dans la revendication 11 ou 12, présentant les étapes suivantes :
utilisation de l'endommagement lié à la réduction de cheveux déterminé ;
sélection d'un agent de traitement pour des cheveux sur la base de l'endommagement lié à la réduction déterminé et la sortie d'informations concernant l'agent de traitement sélectionné.

14. Procédé selon la revendication 13, présentant en outre :
la sélection de l'agent de traitement sur la base d'un résultat de traitement souhaité.

15. Produit programme informatique qui est configuré pour exécuter le procédé selon l'une des revendications 11 à 14 lorsqu'il est exécuté sur un système selon l'une des revendications 1 à 10.
